# EUROPEAN PATENT APPLICATION

(11) **EP 2 474 612 A1**
(43) Date of publication of application: **11.07.2012**
(21) Application number: 11150252.2
(22) Date of filing: 05.01.2011
(51) Int. Cl.: C12N 15/10

(54) **Affinity separation means, and uses thereof to separate, purify or concentrate a target molecule**

(71) Applicant: University College Dublin, National University of Ireland Dublin, Dublin 4 (IE); National Institute for Bioprocessing Research and Training Limited, Dublin 4 (IE)
(72) Inventor: Lee, Gil, D4 Dublin (IE); Muzard, Julien, D6 Dublin (IE)
(74) Representative: Purdy, Hugh Barry

(57) **Abstract**

A method for the purification of a target molecule from a sample containing, or suspected of containing, the target molecule, employs a filamentous phage functionalised magnetic particle in which the filamentous phage is genetically engineered to express at least one coat protein in the form of a fusion protein comprising a foreign peptide having specific binding affinity to a binding partner selected from the target molecule or a surface of the magnetic particle. The method comprising the steps of incubating the affinity separation means with the sample for a period of time sufficient to allow the filamentous phage bind to any target molecule present in the sample, separating the affinity separation means from the sample, and eluting the bound target molecule from the affinity separation means.

## Description

### Field of the Invention

The invention relates methods for the separation, purification and concentration of a target molecule from a sample, and to an affinity separation means.

### Background to the Invention

Conventional immune-chromatography relies on the high-affinity specific interaction between an immunoglobulin and its corresponding epitope. In general, a variable region of immunoglobulin forms a binding pocket that recognizes a specific epitope. In most purification platforms, the probes are polyclonal or monoclonal antibodies. However, polyclonal antibodies obtained from immunized animals are not selective, since they recognize all antigens to which the animal has been exposed in the past. To be used for detection, they should be affinity purified on the columns with the immobilized antigens. Affinity purification of both polyclonal antibodies and monoclonal antibodies dramatically increases their cost.

Genetically-driven bacteriophage, or phage, nanobiotechnology has allowed the development of libraries of diverse nanostructures expressed on the phage surface providing a rich resource of diagnostic, detection and pharmaceutical probes. Phage engineering, which is based on natural mechanisms of selection, amplification and self-assembly, allows directed nanofabrication of bioselective materials with possible applications in gene/drug-delivery, biosensors, nanoelectronics, biosorbents and other areas of medicine, technology and environmental monitoring.

Phage display libraries are obtained by splicing numerous randomized oligonucleotides into individual phages so that each phage displays a unique peptide displayed on the phage surface in multiple copies. This molecular selection technology is more commonly referred to as phage display. Since the discovery of this artificial *in vitro* immune system technology, novel display formats (such as bacterial, ribosome display), library design, and screening strategies are now used routinely in the protein engineering area.

Affinity chromatography in general is an important tool used in protein purifications. The most widely used affinity columns are based on antibody-antigen interactions, however also other ligands such as proteins, receptors are widely used. However, these columns are expensive as the ligands themselves also require prior purification resulting in lot-to-lot variation. Fragility of the ligands reduces the operational life of affinity columns and eventually results in the contamination of final end product with the products degradation.

WO03074548 describes phage ligand sensor devices comprising a sensor-coupled phage having a coat protein that expresses a foreign peptide, and the use of such devices to identity binding ligands of the foreign peptide. Bennett et al (Society for Applied Bacteriology 1997, 83, 259-265) describes a method of detecting the presence of salmonella in a sample by employing a salmonella-specific phage chemically coupled to a magnetic bead.

The present invention attempts to overcome at least some of the above-identified problems.

### Summary of the Invention

The invention broadly relates to a method for the separation, purification or concentration of a target molecule from a sample containing, or suspected of containing, the target molecule, which method employs affinity separation means comprising a solid support coupled to a filamentous phage having specific binding affinity to the target molecule. The method comprises the steps of incubating the affinity separation means with the sample for a period of time sufficient to allow the filamentous phage bind to any target molecule present in the sample, and separation of the affinity separation means from the sample. When the method is for purification or concentration of the target molecule, the method typically includes a step of eluting the bound target molecule from the affinity separation means. The affinity separation means may be pre-prepared prior to the incubation step by coupling the solid support and the filamentous phage, or the solid support and the filamentous phage may incubated together with the sample, wherein the solid support and phage couple together in the incubation mixture.

The invention also provides an affinity separation means comprising a filamentous phage coupled to a solid support by coupling means, the filamentous phage having specific binding affinity for a target molecule.

The invention also provides a kit of parts comprising a solid support and a filamentous phage having specific binding affinity for a target molecule, in which the solid support and/or phage are adapted to allow coupling of the solid support and the phage.

### Brief Description of the Drawings

The invention will be more clearly understood from the following description of an embodiment thereof, given by way of example only, with reference to the accompanying drawings, in which:

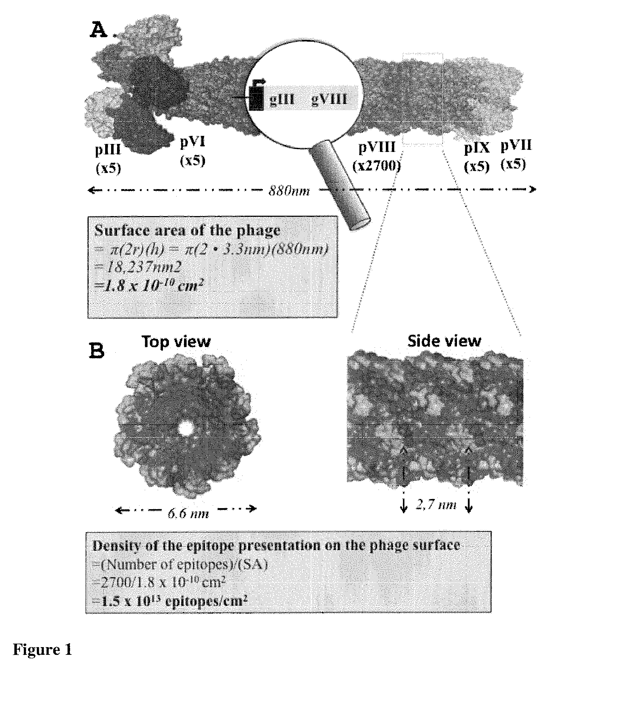

**Figure 1** illustrates the M13 bacteriophage structure. The M13 phage can be easily genetically engineered to display binding-motif at different area (pIII and pVIII) of the phage surface. The calculation of the surface area of the M13 and the density of specific epitopes that can be achieved are detailed in the grey boxes.

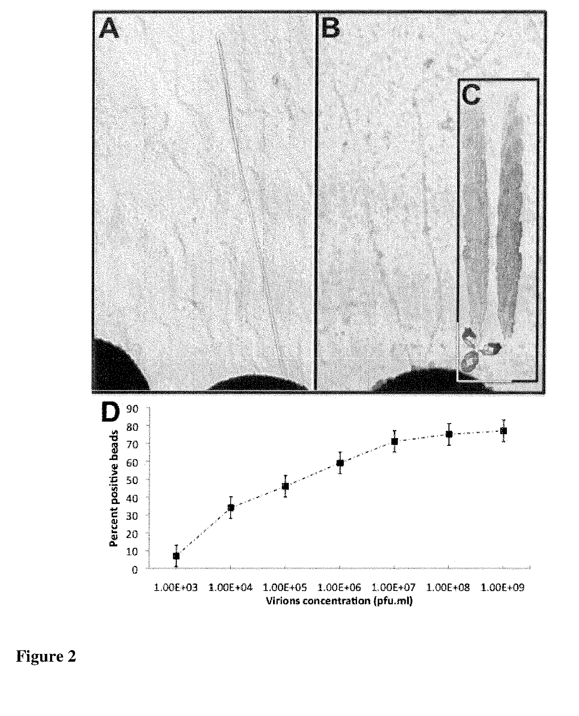

**Figure 2** illustrates an image from transmission electron microscopy (operating at 120 kV) of the filamentous structures bound to paramagnetic particles showing a single (A) and (B) dual captured phages using anti-minor coat phage antibodies. C. Structural interpretation of virion-capture using anti-pIII monoclonal antibody. D. Dose-dependent M13 phage capture on beads. M13 at different concentrations were incubated with a constant amount of superparamagnetic particles for 1 hour. After washing, bacteriophages bound to particles are visualized using anti-pVIII fluorescent antibody and analysed using flow cytometry on a FACScalibur (BD Biosciences).

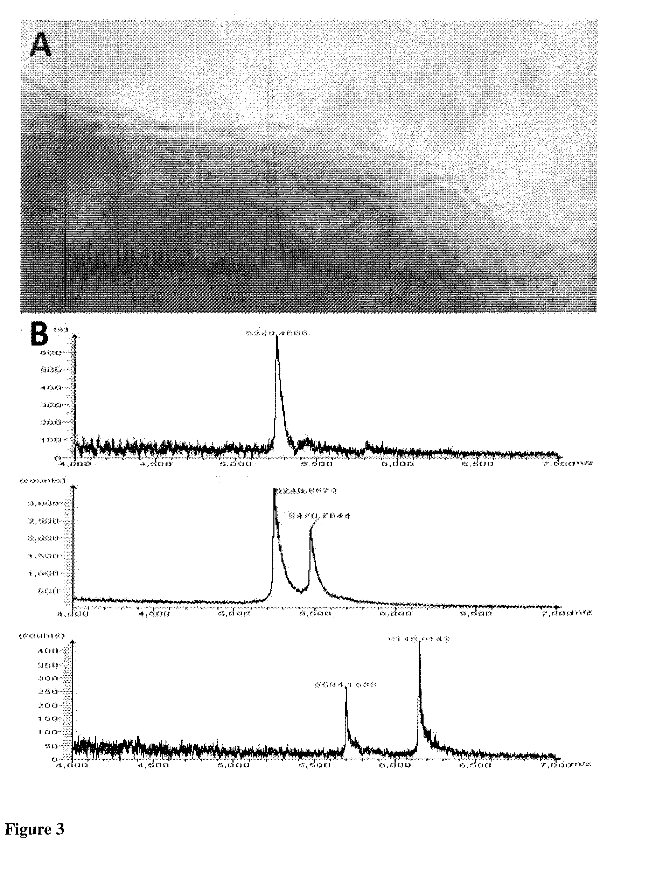

**Figure 3** illustrates A. Visualization under TEM operating at 200 kV showing the surface of paramagnetic beads (1 µm in diameter) functionalized with filamentous phage after negative staining. Phage presence was confirmed using MALDI-TOF MS (overlay). B. Chemical modification screening of the phage coat protein using MALDI TOF showing different states of biotinylation. In the mass spectrum of wild-type M13 bacteriophage, the maximum peak at around 5246 Da corresponds to the molecular weight of native pVIII proteins in M13. The peak at 5470 Da in the middle mass spectrum corresponds to the molecular weight of the chemically modified pVIII proteins. Finally, depending on the experimental conditions (biotinylation kit NHS-biotin or NHS-LC-LC-biotin) a double peak can be observed (lower spectrum panel) indicating that the biotinylation is variable, and can be up to 100% on the pVIII proteins of bacteriophage.

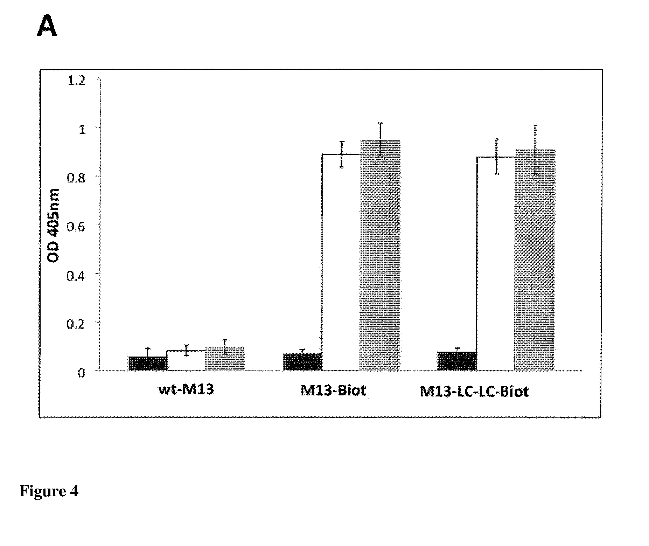

**Figure 4** illustrates an assessment of the binding capacity using direct solid-phase assay (ELISA). BSA (black), streptavidin (blank) and neutravidin (grey) were immobilized on microtiter wells overnight at 4°C. After blocking the non-specific binding sites on the surface, phages (5x10⁷) were applied on the immobilized proteins for 2 hours. Phage detection was performed using HRP labelled anti-phage pVIII antibody followed by ABTS and reading at 450 nm (Biotek Reader).

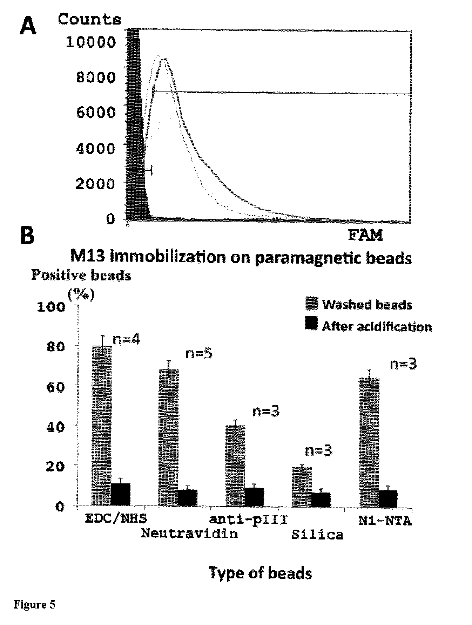

**Figure 5** illustrates a comparison of the efficiency of particles functionalization with virions using flow cytometry. After washing, bound phages are detected using anti-pVIII FITC antibody.

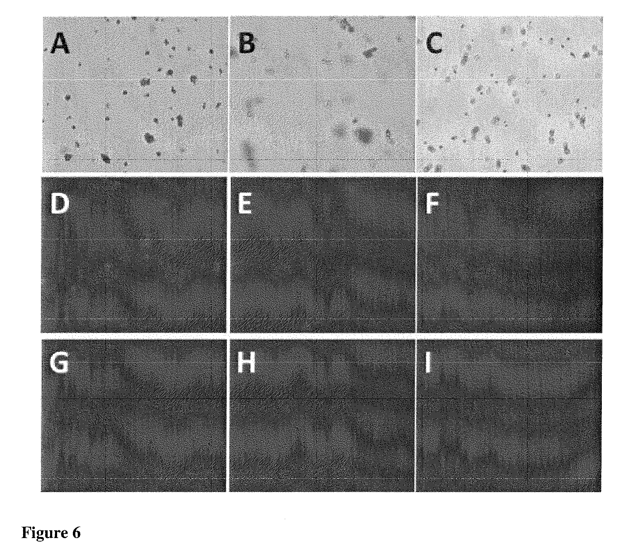

**Figure 6** illustrates one micron superparamagnetic beads functionalized with unmodified wild-type phage (left), anti-minor coat antibody (middle) and chemically biotinylated phage (right). Bright field (A, B and C), fluorescence microscopy of paramagnetic beads before (D, E and F) and after acidification (G, H and I).

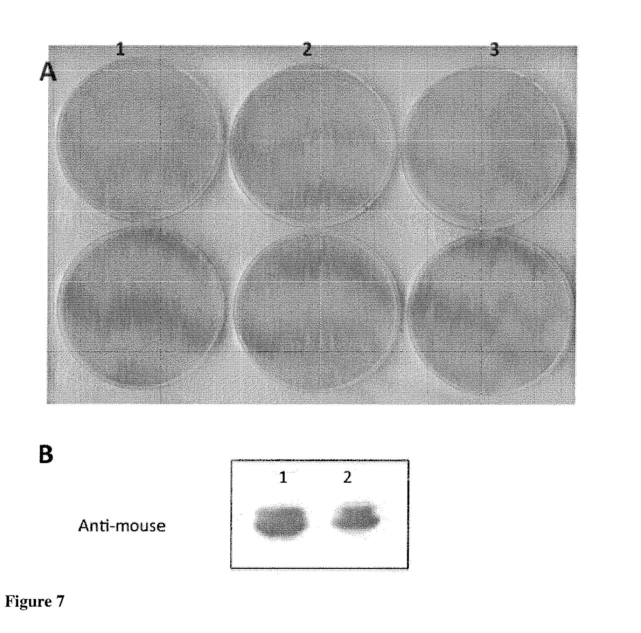

**Figure 7** illustrates the stability and robustness of functionalized beads for two different parameters, namely binding activity and phage leakage, after intensive use of the affinity matrix. (A) Bacterial infection using eluted fractions (Glycine, pH 2.2). No bacteriophages were present in phage-carboxylic particles (1) and biotinylated phage-neutravidin particles (2). Phages were present at a concentration of 10⁶ cfu/mL with the anti-pIII antibody- phage superparamagnetic particles (3). B: Western-blot of the eluted fraction confirming the presence of the anti-phage antibody in the eluted fraction.

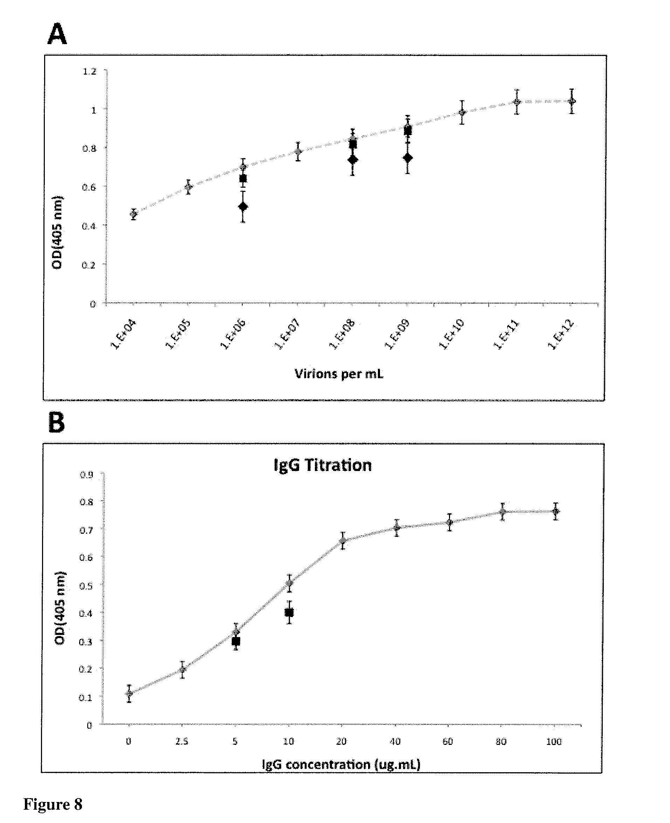

**Figure 8** illustrates the quantification of M13 phage immobilized onto paramagnetic particles using solid-phase assay. M13 phage functionalized paramagnetic particles, at increasing amounts, were incubated with anti-pVIII phage antibody coupled to HRP.

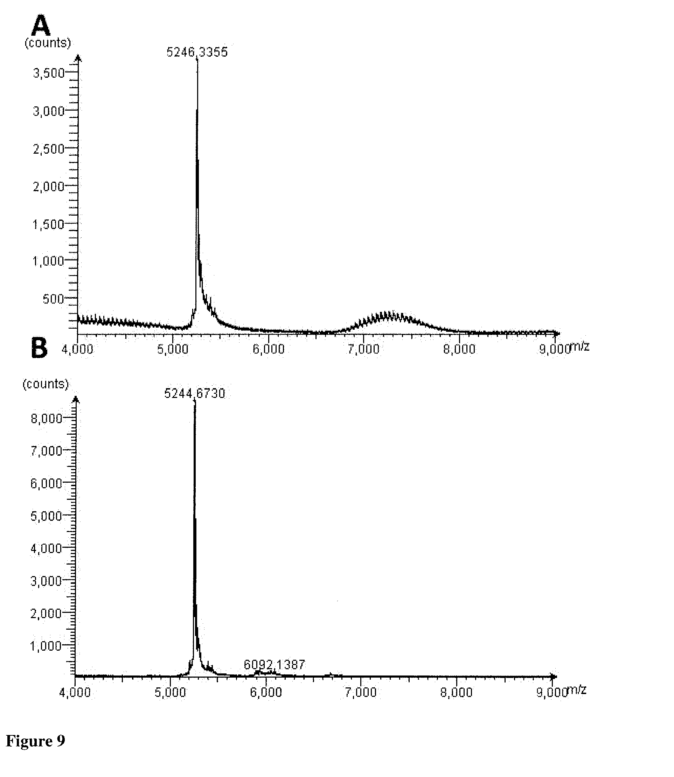

**Figure 9** illustrates MALDI-TOF MS of M13 virus displaying a single modification with (A), poly-ethylene-glycol (PEG-2500) and (B) Dye fluorescent labelling of M 13 with Cy5.

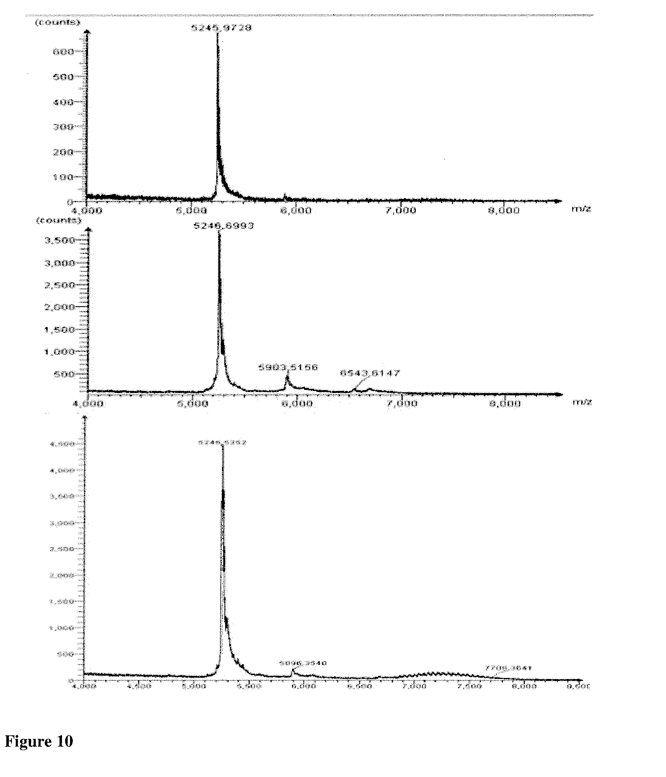

**Figure 10** illustrates top: M 13 virus displaying either silica-binding peptide (top panel) or six-histidine tags in frame with N-terminal end of the pIII protein (not shown). Genetically modified M13 phage, chemically modified with cy5, with wild type pVIII protein, pVIII-cy5 and pVIIII-cy5-cy5 (middle panel), pVIII-cy5-PEG (lower panel).

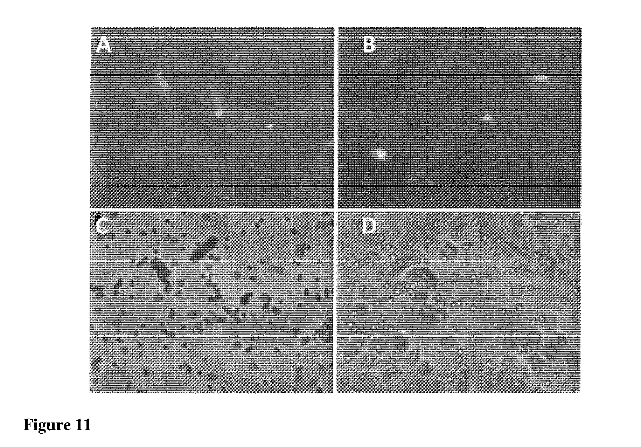

**Figure 11** illustrates immunofluorescence results with (A) silica-binding phage and (B) His-tagged M13 phage immobilized onto silica and NTA coated paramagnetic particles respectively. (C) and (D) correspond to the dark field imaging.

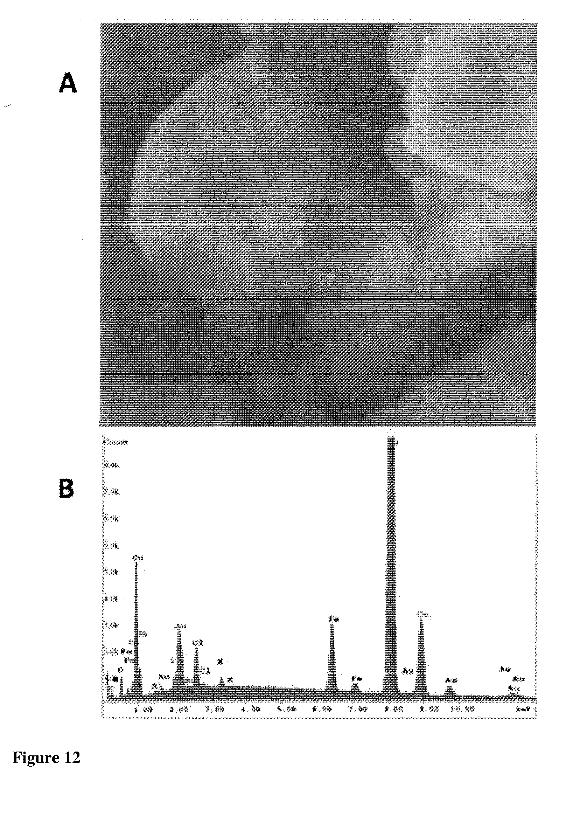

**Figure 12** illustrates (A): Surface scanning electron microscopy (FEG-SEM) confirming the presence of filamentous structures and the binding mode through the tip (pIII) of the virus. (B) EDX spectra on a single particle resulting in the presence of C, H and N atoms from the M13 virus. Samples were coated with gold. Low-resolution image showing the magnetic lines of the preparation dried under magnetic field.

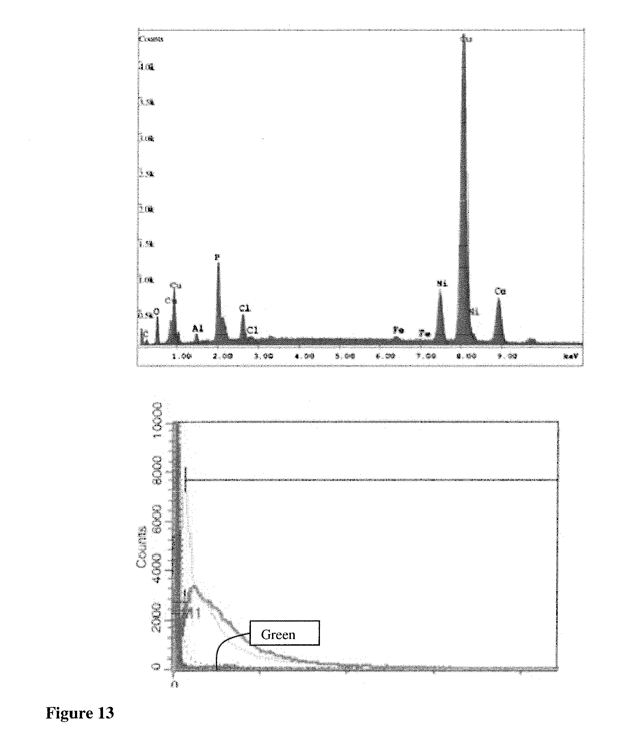

**Figure 13** illustrates (Upper panel) EDX on NTA particles. Ni⁺⁺ on the particles surface PEG-NTA coated is identified using the EDX. (Lower panel) FACS detection using anti-pVIII-FITC on superparamagnetic particles. Indicated histogram corresponds to an example of fluorescence intensity from magnetic particles functionalized with silica or histidine tagged binding bacteriophages.

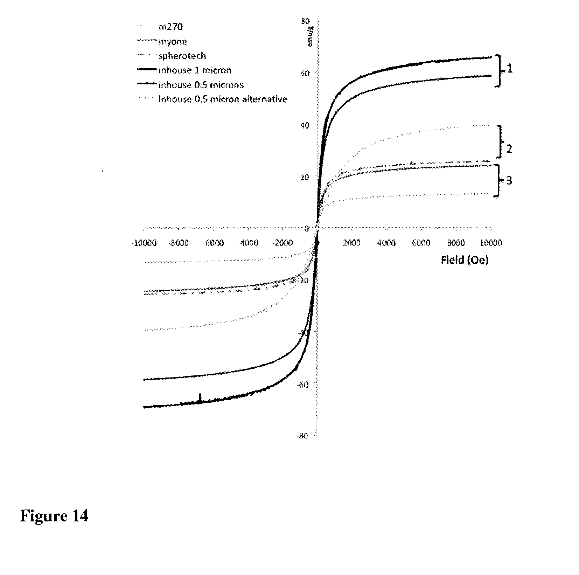

**Figure 14** illustrates the stronger magnetic behaviours of the material used for this demonstration compared to m270, myone (Dynabeads, Invitrogen) and magnetic particles from Spherotech. The higher the force the quicker and easier it is to remove the particles from solution, with 1/: In house particles have a higher saturation of magnetisation, 2/: Control of the core material gives tuneable magnetic properties, and 3/: Common commercial particles.

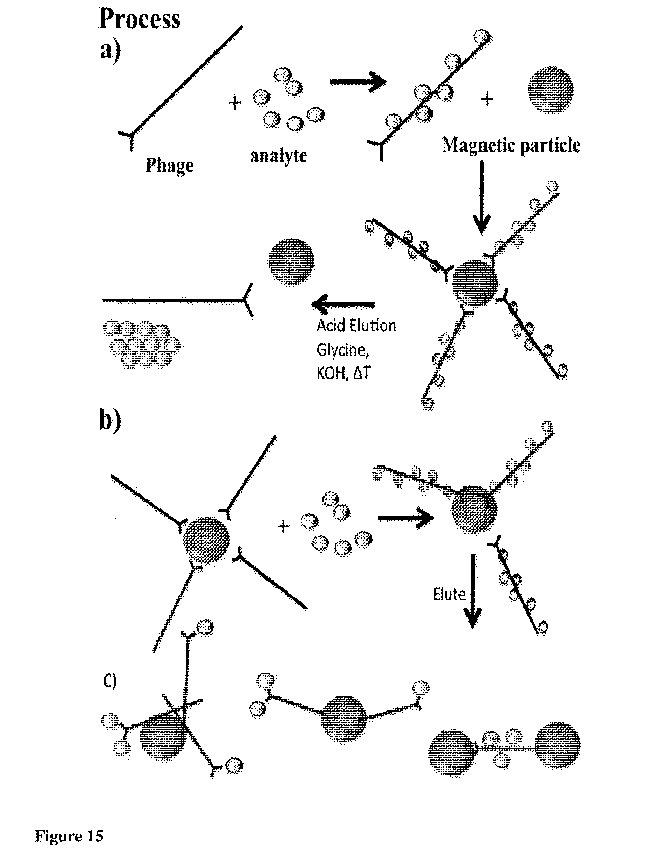

**Figure 15** illustrates the summary of the mean process discloses in the present invention. A) Phage particles harbouring a chemically and/or genetically engineered affinity motif through one tip are incubated with the target molecule (i.e. therapeutic antibody or antibody fragments) to be purified. Phages bound to the target molecule are then capture using superparamagnetic particles. After washing, the analyte can be eluted from the solid support and separated from the whole phage particles in a one step. B) The analyte to be purified is incubated with superparamagnetic particles functionalized with bacteriophages and the eluted from the solid support. Binding occurs by the pVIII major coat protein C) Alternative configurations of phages bound to superparamagnetic particles and analyte binding mode.

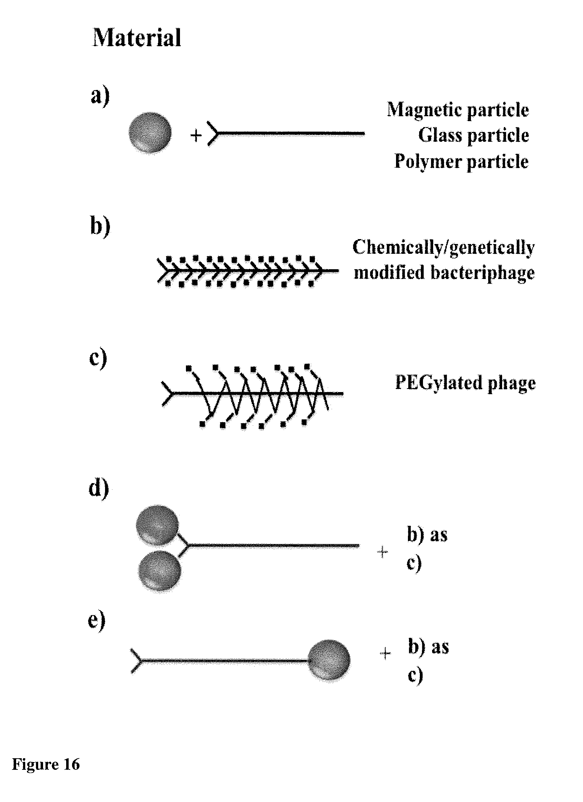

**Figure 16** illustrates the material proprieties disclose in the present invention. a) a superparamagnetic particle and a bacteriophage, b) Modified bacteriophage on the pVIII surface ; c) PEGylated phage ; d) phage-particles complexe and e) Paramagnetic particle functionalized by phage proteins

### Detailed Description of the Drawings

The invention provides an affinity separation means comprising a filamentous phage coupled to a solid support by coupling means, the filamentous phage having specific binding affinity for a target molecule, and the use thereof in the separation, purification or concentration of a target molecule from a sample.

The solid support acts as a support for the phage, and can take a number of different forms including a mobile form (for example magnetic or non-magnetic beads or particles) or a stationary form (for example a microtitre plate, a filter or membrane, or a stationary phase of a chromatography column). One example of a mobile solid support is beads or particles functionalised with the filamentous phage. Suitably, the solid support is a magnetic particle, preferably a paramagnetic particle, and ideally a superparamagnetic particle. If the solid phase comprises magnetic particles that are functionalized with phage, the total protein that can be bound to the surface of the hybrid phage-particle material will increase by as much as two orders of magnitude. This enables reasonable levels of recovery of target molecules. In the method of the invention, the affinity separation means is suitably separated from the sample by application of a magnetic field. Other types of beads include sepharose or agarose beads. The solid phase may be nano- or micro- scale particles. Preferably, the magnetic beads are nanobeads.

The solid support may also take the form of a stationary phase of a chromatographic column. When the solid support is a stationary phase of a chromatography column, separation of the affinity separation means from the sample generally means allowing the sample to run through the column. Examples of stationary phases include organic material selected from the group consisting of agarose, dextran, cellulose, chitosan, polyacrylamide, polyacrylate, polystyrene, polyvinyl alcohol, polyethylene glycol, and combinations thereof. Other solid supports applicable for the present invention include plates, for example microtitre plates, and filters, membranes, semi-permeable membranes, a capillary columns, a microarrays, and a multiple-well plate.

The term "filamentous phage" should be understood to mean phage in which the virion DNA is encased within a tubular sheath, typically about 6nm in diameter, made of thousands of subunits of the major coat protein pVIII (g8p) and a smaller number of copies of minor coat proteins for example pIII (g3p), pVI (g6p), pVII (g7p), and pIX (g9p). Preferably, the phage is a Ff class of filamentous bacteriophage (f1, fd or M13). Ideally, the phage is M13. Typically, the phage is genetically engineered to express at least one (or two, three, four or five) coat protein in the form of a fusion protein comprising a foreign peptide (or polypeptide) having specific binding affinity to a binding partner. Ideally the foreign peptide has from 5 to 50 amino acids. Typically the coat protein is selected from pVIII, pIII, pVI, pVII, and pIX. Generating recombinant phage that express foreign peptides at their surface is well known in the art, and is described in US5427908, US5403484, US5432018, US270170, and Petrenko et al (1996) Protein Engineering 19(6): 797-801.

In a preferred embodiment, the foreign peptide expressed as part of a hydrid coat protein has specific binding affinity for the target molecule.

In a different embodiment, the foreign peptide has binding affinity for a binding partner which is not the target molecule, for example a binding partner that forms part of the solid phase. Thus, for example, the foreign peptide may be a silica-binding peptide. In this case, the solid phase may be functionalised with silica, for example solid phase beads, particles or plate coated with silica, wherein the filamentous phage is coupled with the solid phage by virtue of specific binding between the silica-binding peptide on the phage and the silica coating on the solid phase. Similarly, the foreign peptide may be a polyhistidine tag, in which case the solid support suitably comprises, or is functionalised or coated with a binding partner for the poly-His peptide, for example, nickel or cobalt, and wherein the filamentous phage is coupled to (or capable of being coupled to) the solid support by means of a specific binding reaction between the polyhistidine tag forming part of the filamentous phage and, for example, the nickel or cobalt forming part of the solid support.

In one preferred embodiment of the invention, the phage is genetically modified to express at least two (different) coat proteins as recombinant fusion proteins, each comprising a different foreign peptide. Typically, one coat protein (for example pVIII) is expressed as a fusion protein comprising a foreign peptide having specific binding affinity for the target molecule, and another coat protein (for example pIII or any other coat protein) is expressed as a fusion protein comprising a foreign peptide (for example a silica-binding peptide or a polyhistidine tag) having specific binding affinity for a binding partner which is part of the solid phase. Alternatively, one coat protein (for example pVIII) is expressed as a fusion protein comprising a foreign peptide having specific binding affinity for a first target molecule, and another coat protein (for example pIII) is expressed as a fusion protein comprising a foreign peptide having specific binding affinity for a second target molecule. Alternatively, one coat protein (for example pVIII) is expressed as a fusion protein comprising a foreign peptide having specific binding affinity for a first part or component of the solid phase, and another coat protein (for example pIII) is expressed as a fusion protein comprising a foreign peptide having specific binding affinity for a second part or component of the solid phase.

In one embodiment, the solid support is coupled to the filamentous phage by means of a coupling ligand. Typically, the coupling ligand is an antibody that is coupled to the solid support (ideally chemically coupled or coupled by means of conjugation) and has specific binding affinity for a coat protein of the filamentous phage. Polyclonal and monoclonal antibodies having specific binding affinity to filamentous phage coat proteins, especially pIII, pVIII and pVI, are known in the art [NEB - E8033S ; Pierce - MA1-06603, MA1-06604 ; Sigma, B2661 B7786 ; AV19003 - GE Healthcare 279421-01 ; Aviva Systems Biology, AVARP19003_T100 ; Santa-Cruz Biotechnology Inc, sc53004 ; sc65380 ; - Abcam ab20337 ; ab6188 ; ab50370 ; ab24229 ; ab17269 ; ab9223 and ab92225...]. Coupling of the antibody to the solid phase may be achieved using any method known in the art, for example ethyl-dimethyl-aminopropylcarbodiimide and N-hydroxy-succinimide (EDC/NHS) cross-linking (or PEG-EDC/NHS coupling chemistry) which, for example, couples basic amino acid residues on the antibody with the solid support, or biotin-biotin binding partner coupling where, for example, the surface of the solid support is functionalised with a biotin binding partner and basic amino acids of the antibody are biotinylated.

In another embodiment, the solid support is covalently (chemically) coupled to the filamentous phage directly. Coupling of the filamentous phage to the solid phase may be achieved using any method known in the art, for example ethyl-dimethyl-aminopropylcarbodiimide and N-hydroxy-succinimide (EDC/NHS) cross-linking (or PEG-EDC/NHS coupling chemistry) which, for example, couples basic amino acid residues on a coat protein with the solid support (ie. PEI-PEG-NH2), or biotin-biotin binding partner coupling where, for example, the surface of the solid support is functionalised with a biotin binding partner and basic amino acids of a phage coat protein are biotinylated.

As indicated above, the solid support may selected from the group consisting of a bead, a particle (i.e. magnetic, glass or polymeric, or a mixture thereof), sepharose, a membrane, a semi-permeable membrane, a capillary column, a microarray, and a multiple-well plate.

Typically, the solid support comprises an inorganic material layer, suitably selected from the group consisting of glass, alumina, silica, zirconia, magnetite, a semiconductor, and combinations thereof.

Suitably, the solid support comprises an organic material, typically selected from the group consisting of sepharose, agarose, dextran, cellulose, chitosan, polyacrylamide, polyacrylate, polystyrene, polyvinyl alcohol, polyethylene glycol, and combinations thereof.

The target molecule may be any biological molecule including but not limited to a peptide, polypeptide, protein, protein complex, recombinant protein, antibody (or a fragment of an antibody), for example an immunoglobulin selected from the group IgG, IgM, IgA, IgD, IgE, chimeric IgG, human IgG, humanised IgG, polynucleotide, sugar, polysaccharide, or an organism, for example a bacteria or virus. In one embodiment, the term "target molecule" excludes organisms. The sample may be a biological fluid, for example blood, blood plasma, blood serum, cerebrospinal fluid, urine or a non-biological fluid such as a man-made mixture of components, for example a product stream obtained from a fermentation process or an aqueous cell culture, or an environmental sample.

In another embodiment, the filamentous phage are functionalised with a hydrophilic polymer such as for example PEG, typically via a phage coat protein. It has been widely demonstrated that the conjugation of a hydrophilic polymer such as PEG to many molecules can significantly increases their half-life and/or their solubilisation. Hydrophilic polymers such as PEG provide a more stable conformation and increases the size and the weight of the phage. The biological stability will increase because it will be impossible for proteins to reach the surface of the coat proteins that have been functionalized, which will make them less susceptible to enzymatic attack. Second, their chemical stability will increase because the hydrophilic polymers will decrease the concentration of chemicals at the surface of the phage. The functionalization of the phage with PEGs (or other hydrophilic polymers) will allow the anlayte that has been captured on the phage to be released under less harsh conditions and will allow the ligands expressed on their surface to be more fully regenerated.

Functionalisation of the phage with a hydrophilic polymer may be accomplished by known methods in the art. For example, polyethylene glycol may be attached to the protein either directly or by an intervening linker. One non limitative way of proceeding is to covalently bind polyethylene glycol through amino acid residues via a reactive group, such as a free amino or carboxyl group. Linkerless systems for attaching polyethylene glycol to proteins are described in Delgado et al., Crit. Rev. Thera. Drug Carrier Sys. 9:249-304 (1992), Francis et al., Inter J. of Hematol. 68:1-18 (1998), US 4,002,531 , U.S. 5,349,052, WO 95/06058 and WO 98/32466. One system for attaching polyethylene glycol directly to amino acid residues of proteins without an intervening linker employs tresylated MPEG. Upon reaction of protein with tresylated MPEG, polyethylene glycol is directly attached to amine groups of the protein. Thus, phage employed in the present invention can include phage-polyethylene glycol conjugates, for example those produced by reacting phage coat proteins with a polyethylene glycol molecule having a 2,2,2-trifluoreothane sulphonyl group. Polyethylene glycol can also be attached to proteins using a number of different intervening linkers. For example, US 5,612,460 discloses urethane linkers for connecting polyethylene glycol to proteins. Protein-polyethylene glycol conjugates wherein the polyethylene glycol is attached to the protein by a linker can also be produced by reaction of proteins with compounds such as MPEG- succinimidylsuccinate, MPEG activated with 1 ,1'-carbonyldiimidazole, MPEG- 2,4,5-trichloropenylcarbonate, MPEG-p-nitrophenolcarbonatβ, and various MPEG- succinate derivatives. A number additional polyethylene glycol derivatives and reaction chemistries for attaching polyethylene glycol to proteins are described in WO 98/32466. The number of polyethylene glycol moieties attached to each phage (i.e., the degree of substitution) may also vary.

In a preferred embodiment, the invention relates to a method of separating a target molecule from a sample containing, or suspected of containing, the target molecule, which method employs an affinity separation means comprising a filamentous phage coupled to a solid support (typically a magnetic particle or bead), wherein the filamentous phage has specific binding affinity for the target molecule, and wherein the filamentous phage is genetically engineered to express at least one coat protein as a fusion protein comprising a foreign silica-binding peptide, and wherein the solid support comprises a silica coating, and wherein the phage is coupled to the solid support by means of the silica-binding peptide of the phage binding to the silica coating on the solid support, the method comprising the steps of incubating the affinity separation means with the sample for a period of time sufficient to allow the filamentous phage bind to any target molecule present in the sample, and separation of the affinity separation means from the sample, and optionally eluting the bound target molecule from the affinity separation means.

The invention also relates to an affinity separation means comprising a filamentous phage coupled to a solid support, typically a magnetic particle or bead, wherein the filamentous phage has specific binding affinity for a target molecule, and wherein the filamentous phage is genetically engineered to express at least one coat protein as a fusion protein comprising a foreign silica-binding peptide, and wherein the solid support comprises a silica coating, and wherein the phage is coupled to the solid support by means of the silica-binding peptide of the phage binding to the silica coating on the solid support.

In another embodiment, the invention relates to a method of separating a target molecule from a sample containing, or suspected of containing, the target molecule, which method employs an affinity separation means comprising a filamentous phage coupled to a solid support, typically a magnetic particle or bead, wherein the filamentous phage has specific binding affinity for the target molecule, and wherein the filamentous phage is genetically engineered to express a first coat protein as a fusion protein comprising a first foreign peptide having specific binding affinity for a coupling ligand, and wherein the solid support comprises or is functionalised with the coupling ligand, and wherein the phage is coupled to the solid support by means of the first foreign peptide of the phage binding to the coupling ligand on the solid support, and wherein the filamentous phage is genetically engineered to express a second coat protein as a fusion protein comprising a second foreign peptide having specific binding affinity for the target molecule, the method comprising the steps of incubating the affinity separation means with the sample for a period of time sufficient to allow the filamentous phage bind to any target molecule present in the sample, and separation of the affinity separation means from the sample, and optionally eluting the bound target molecule from the affinity separation means.

The invention also relates to an affinity separation means comprising a filamentous phage coupled to a solid support, typically a magnetic particle or bead, wherein the filamentous phage has specific binding affinity for the target molecule, and wherein the filamentous phage is genetically engineered to express a first coat protein as a fusion protein comprising a first foreign peptide having specific binding affinity for a coupling ligand, and wherein the solid support comprises or is functionalised with the coupling ligand, and wherein the phage is coupled to the solid support by means of the first foreign peptide of the phage binding to the coupling ligand on the solid support, and wherein the filamentous phage is genetically engineered to express a second coat protein as a fusion protein comprising a second foreign peptide having specific binding affinity for the target molecule,

In a further embodiment, the invention relates to a method of separating a target molecule from a sample containing, or suspected of containing, the target molecule, which method employs an affinity separation means comprising a filamentous phage coupled to a solid support (typically a magnetic particle or bead) by means of a coupling antibody, wherein the coupling antibody has specific binding affinity for a phage coat protein (i.e. it is anti-phage coat protein antibody, for example an anti-pVIII or anti-pIII monoclonal antibody) and is chemically or physically coupled to the solid support, the method comprising the steps of incubating the affinity separation means with the sample for a period of time sufficient to allow the filamentous phage bind to any target molecule present in the sample, and separation of the affinity separation means from the sample, and optionally eluting the bound target molecule from the affinity separation means.

The invention also relates to an affinity separation means comprising a filamentous phage coupled to a solid support (typically a magnetic particle or bead) by means of a coupling antibody, wherein the coupling antibody has specific binding affinity for a phage coat protein (i.e. it is an anti-phage coat protein antibody, for example an anti-pVIII or anti-pIII monoclonal antibody) and is chemically or physically coupled to the solid support.

The invention also relates to an affinity separation means comprising a magnetic particle or bead coupled to a phage by means of a coupling antibody, wherein the coupling antibody is selected from an anti-pVIII, anti-pIII, anti-pVI, anti-pVII and anti-pIX monoclonal antibody, wherein the antibody is chemically or physically coupled to the solid support.

In one embodiment, phage comprises a label that can be directly detected. By "directly detected" is meant that the bacteriophage can be labelled in advance and still bind its target upon addition to a cell/sample, etc. containing the target for direct detection via the label on the bacteriophage, i.e., no secondary antibody is necessary. Furthermore, by "label" is meant a means for visualization, such as a recognition site for direct phosphorylation, biotinylation, chemical linkages, etc. engineered into the virus, or such as a directly visualized label requiring no chemical reaction to detect, e.g.,, the virus expresses a fluorescent protein or is labeled by a radioactive moiety. Phage can be modified to include a label in advance, then bound to the phage target in a sample, and visualized to detect the presence and localization of the bound phage. Visualization may or may not require a chemical reaction. Thus, directly detected does not require interaction with a secondary labelled ligand, for example an antibody. An example of a fluorescent protein is the green fluorescent protein (GFP).

Examples of detectable and reporter substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin. Suitably, the reporter molecule is a fluorescent protein (GFP, YFP). Typically, the reporter molecule is an enzyme such as HRP, phosphatase alkaline, enterokinase. In another embodiment, the reporter molecule is a selected from a peptide, a toxin, a fragment from a large protein, and biotin. Typically, the reporter molecule is an affinity tag, such a Strep Tag, Strep Tag II, Avi Tag, His Tag, SA binding peptide, T7 Tag epitope. Alternatively, the reporter molecule is a radioactive compound.

The invention also relates to a filamentous phage that is genetically engineered to express at least one coat protein in the form of a fusion protein comprising a foreign peptide having specific binding affinity to a binding partner, in which the binding partner forms part of a solid support.

Typically, the foreign peptide is a silica-binding peptide, and the solid support comprises, or is functionalised or coated with, silica, and wherein the filamentous phage is coupled to the solid support by means of a specific binding reaction between the silica-binding peptide forming part of the filamentous phage and the silica forming part of the solid support.

Suitably, the peptide is a polyhistidine tag, and the solid support comprises, or is functionalised or coated with, nickel or cobalt, and wherein the filamentous phage is coupled to the solid support by means of a specific binding reaction between the polyhistidine tag forming part of the filamentous phage and the nickel or cobalt forming part of the solid support.

Preferably, the filamentous phage is genetically engineered to express (a) a first major coat protein in the form of a fusion protein comprising the major coat protein and a peptide having specific binding affinity to the target molecule, and (b) a second major coat protein in the form of a fusion protein comprising the major coat protein and a peptide having specific binding affinity to a binding partner forming part of the solid support.

Ideally, the filamentous phage is pegylated.

The invention also relates to a solid support coupled to a coupling antibody, wherein the coupling antibody has specific binding affinity for a phage coat protein (i.e. an anti-phage coat protein antibody, for example an anti-pVIII or anti-pIII monoclonal antibody). Typically, the antibody is chemically or physically coupled to the solid support.

### EXPERIMENTAL:

### Vectors and cells

M13KE is a derivative of M13mp19 modified, using standard molecular biology, for expression of peptides as N-terminal pIII as pentavalent display on the virion surface. E. *coli* strain ER2738 (genotype: F'proA+B+ 1acIq Δ(lacZ)M15 zzf::Tn10(TetR)/ fhuA2 g1nV Δ(1ac-proAB) thi-1 Δ(hsdS-mcrB)5) is grown in Luria Bertani (LB) medium at the temperature of 37°C overnight under orbital shaking at 50 rpm. The *E.coli* strain is an *fhuA2* version of *E. coli* NM522 in which the F' can be selected for using tetracycline. *E. coli* strain TG1 (genotype: F'traD36 1acI^{q} Δ(1acZ) M15 proA⁺B⁺ /supE Δ(hsdM-mcrB)5 (rₖ- mₖ- McrB⁻) thi Δ(1ac-proAB). E. coli strain XL1- blue (genotype: recA1 endA1 gyrA96 thi-1 hsdR17 supE44 re1A1 1ac [F' proAB 1acIqZΔM15 Tn10 (Tetr)]. The stock solutions of E. coli-M13KE is stored at - 80°C in 50% glycerol (Freezer Bionanosciences UCD room-A1-CSCB-New Brunswick U570 BOX 1 G1 - 3 box JM1).

### General plaque formation and phage titer experiment:

After a series of 10 fold dilutions in microtiter plates, 5 µl of M13KE wild type and recombinant M13KE phages were plated on LB Agar plates with a ER2738 cell lawn supplemented with 20 µg/ml IPTG, 20 µg/ml XGal, 12 µg/ml tetracycline. The plates were incubated at 37 °C overnight.

### Large scale amplification of M13.

M13 virus was grown and purified following standard bacteriology and biochemical protocols. Briefly, a 1 L of an *E*. *coli* ER2738 culture was grown in LB-tet media to mid-log phase and infected with 1 mL of wild-type M13 bacteriophage (10¹² PFU/ mL). The culture was incubated at 37 °C with shaking for 5 6 h, centrifuged to remove bacterial cells, the virus collected by PEG NaCl (20% PEG and 2.5 mol/L NaCl) precipitation and reconstituted in Tris buffer. The typical yield was 30 mg of M13 per liter of infected bacteria suspension. The phage concentration was estimated spectrophotometrically using an extinction coefficient of 3.84 cm²/mg at 269 nm.

### Surface modification on phage

M13 phages were grown in *E.coli,* purified and subjected to *in vitro* biotinylation by using either the NHS-LC-LC-biotin (Pierce 21343) or the NHS-Biotin (Pierce 20217) kit according to the manufacturer's protocol (Pierce, Rockford, IL). Biotinylated particles were then dialyzed against PBS overnight to remove free biotin, and store until further use. Presence of biotin on the phage surface was confirmed by the HABA method (Sigma H2153). PEG-2500 Da, and fluorescent dye (Cy5, Amersham, PA25031) were chemically grafted onto the M13 surface using amine chemistry.

### Assessment of the ability of avidin, streptavidin and neutravidin to bind Biot-phages

Biotinylated-phages were detected by using a both dot blot and quantitative solid phage assay. Aliquots of BSA, avidin, streptavidin and neutravidin were bound to well of a microtitration plate. After immobilization, the free binding sites were blocked with PBS containing 2% skim milk, washed and incubated with phage solutions for 2 hours at room temperature. Binding of the phage to the immobilized protein was detected with anti-phage- HRP (Amersham, UK) were developed by using a peroxidase substrate (Pierce 34028)

In case of dot blotting, various amounts of modified phages were blotted on PVDF membranes according to the manufacturer's recommendations. The membranes were washed once in PBS, blocked with MPBS, and incubated with HRP-conjugated anti-M13 antibody, which had been diluted 5000 times, or with streptavidin-HRP for one hour at room temperature, and the detection was performed using the precipitating TMB substrate (Pierce, 34018) as described above.

### MALDI-TOF MS of M13 subunit.

A solution of M13 virus (1 mg/mL) was directly spotted onto a MALDI plate using Millipore ZipTip µ-C18 ® tips to remove the salts. The mass spectrum of the major phage protein was acquired using a SAI MALDI-TOF mass spectrometer (SAI, UK) with either MS grade sinapinic acid or alpha cyano 4 hydroxycinnamic acid in 70% acetonitrile and 0.1 % TFA as the matrix, and operating in linear mode.

### Home made preparation of 2 - 5 microns magnetic particles-1L scale

The reaction is under N2 atmosphere with vigorous stir. Bubble N2 into 500 ml water for 1hr to remove 02. Dissolve 48g FeCl2·4H2O and 98g FeCl3·6H2O in 250 ml deoxygen water in a 1L three neck under N2 atmosphere. Leave the flask on ice bath. Add 200 ml NH3·H2O rapidly with vigorous stir. Leave on ice bath for 45 min. Heat the solution to 85 °C (outside temp. 120°C) for 1 hr. Add 30ml oleic acid and heated for another 1hr. Cool to room temperature. Transfer the slur to a 600ml beaker. Wash the slur with 200ml ethanol for three times. Each time the black magnetite was collected using the block magnet. Wash the slur with 200 ml water for three times. Wash the slur with 200 ml 20% perchloric acid for three times to dissolve Fe(OH)2 and Fe(OH)3. Wash the slur with 200ml water for three times. The particle is highly hydrophobic in this step. Wash the slur with 200ml ethanol for three times. Add 350 ml hexane to the particle. The particle can be well dispersed in this step. Distribute the particle into 100 ml glass bottles. Store the particle at 4 °C. Clean the glassware with RBS. The remaining black stain can be removed using aqua regia. In the next day, remove water from the nanoparticle solution. Use a magnet to pull the solution to one side of the bottle. The clear water layer is visible at the bottom of the bottle. Pipette out the water. Measure the particle concentration. Weigh the small glass dish. Add 1ml particle solution to it. Record the weight. Air dry the hexane. Measure the weight again. Report the particle percentage on weight percentage. The particle concentration should be between 6-13% (60-130mg/ml).

Commercially particles, such as Dynabeads (Invitrogen) were used in our work-package demonstration. However, considering their oxidation state, their poor magnetic characteristics and the autofluorecence behaviors, we decided to use and focus on in-house superparamagnetic particles (MagSense Inc.)

### Phage immobilization on the magnetic particles: Preparation of phage coated magnetic particles.

The paramagnetic particle (coated with surface was functionalized with filamentous M13 phage using: (A) EDC/NHS coupling chemistry (Pierce, 1-Ethyl-3-[3-dimethylaminopropyl]carbodiimide hydrochloride); (B) biotinylated phages (both, Pierce, EZ-Link-NHS-Biotin and EZ-Link) (-neutravidin coated particles); (C) anti-pIII M13 coat-protein antibody (New England Biolabs); (D) Genetically modified M13 displaying either silica binding peptide and a six histidine motif onto pIII protein were incubated with either silica coated particles or -PEG-NTA-Ni²⁺ superparamagnetic particles (in house synthesis)

The phage preparation was incubated on a rotation wheel for 2 hours or overnight with the superparamagnetic particles. The density of phage on the solid support (ie. superparamagnetic particles) was determined with an enzyme linked colorimetric immunoassay with a comparison with a M13 calibration curve. The particles were washed with a phosphate buffered saline (PBS) three times and then the monoclonal anti-pVIII IgG horseradish peroxidase (HRP) conjugate was added to the particles at 1:100 dilution ratio. After incubation for 1 hour, the particles were washed with PBS-Tween three times to remove the excess phage and store at 4C for further characterization.

### Preparation of samples for Electron Microscopy analysis

A solution of magnetic particles functionalized with filamentous virions (20 µL) was deposited onto a 300-mesh carbon-coated copper grid for 2 min. The grid was then stained with 2% uranyl acetate before TEM analysis. Either a JEOL 2100 operating at 200kv or a FEI Tecnai 120 was used to characterize the different sources of magnetic particles functionalized with filamentous virions. Field Emission Scanning Electron Microscopy was carried out directly on the gold coated particles functionalized with M13 viruses.

### Fluorescence imaging

Five µL of wt M13 phages (5.10⁷ cfu), chemically or genetically modified phages (5.10⁷ cfu) were incubated with 50 µl of anti-M13 either FITC or PE at room temperature in dark. After incubation, the beads were washed and the mixtures were mounted on glass slides, covered with cover glasses, and viewed through a confocal laser-scanning microscope (Leica TCS NT, Heidelberg, Germany).

### Magnetic separation of antibody based on magnetic phage-particles

The magnetic-phage particles were incubated at room temperature for 16h with anti-phage coat proteins monoclonal antibodies. Magnetic beads were concentrated using a permanent magnet, washed 3 times in sterile PBS-Tween, and the bound antibodies were recovered using low pH elution buffer. The eluted fractions were immediately neutralized and then dialysed against PBS and submitted to protein quantification using the BCA assay (Pierce, 23227) and Western-blotting.

### Target Recovery

In order to measure the recovery of magnetic phage particles, different amounts of mouse anti-phage antibody were applied to the selected phage column. The amount of anti-M13 that completely bound to the column was used as a measure of the recovery. Fifty micrograms of anti-M13 was applied to a magnetic phage column. After washing the column with PBS, the bound anti-phage was eluted with either 1M NaCl or with pH 2 Glycine buffer and the eluted fraction was collected. The amount of protein in the eluate fraction was determined using the micro-BCA assay as describe above.

Absorbance at 630 nm was measured on a BIO-Tek ELISA reader.

### Robustness of the phage column - stability over storage

The robustness of the column was checked by multiple cycles of loading, washing, elution and re-equilibration. Each time one hundred micrograms of anti-M13 was applied to the column phage column and eluted in the same way as the recovery experiment. From different cycles, the amount of protein was determined in eluted fraction to check the robustness of the column after intensive use. The eluate fraction was used to infect *E. coli* cells as described previously. Briefly, a 10-fold dilution series, 10⁻¹ to 10⁻⁹, from the eluate fractions were made in sterile Luria Broth Base medium (LB) and used to infect *E.coli* TG-1 cells for 30 min at 37C. Cell suspensions were plated on agar plates containing 10 µg/ml tetracycline and incubated overnight at 37°C. Both Infected and non-infected cells were used as controls. After incubation, colonies infected with phages were counted. To control the purity of the eluted fraction from the phage columns, elution solution of both NaCl and low pH peak fractions were concentrated 20 times using Vivaspin-15 concentrators (molecular weight cut-off 10 000). Concentrated samples were applied on 10% SDS gels for electrophoresis and stained with Coomassie Brilliant blue and submitted for Western blotting using anti-phage antibody.

### Western blotting analysis

Both precipitant and concentrated supernatant from the previous reaction were electrophoresed by 10 % SDS-PAGE gel and transferred to a nitrocellulose membrane (semidry 20 V, 20 min). The membrane was blocked with 5% skim milk in 1xPBS at 4 °C overnight and incubated with a 1/1000 dilution of HRP-anti-mouse IgG at room temperature for 2 hours. The membrane was washed and then probed with 1/10 000 dilution of anti-mouse IgG-Alkaline phosphatase conjugate (Sigma). The membrane was washed 6 times with PBST (PBS, 0.5% Tween-20), developed with NBT/BCIP reaction.

The invention is not limited to the embodiments hereinbefore described which may be varied in construction and detail without departing from the spirit of the invention.

## Claims

1. A method for the purification of a target molecule from a sample containing, or suspected of containing, the target molecule, which method employs a filamentous phage functionalised magnetic particle in which the filamentous phage is genetically engineered to express at least one coat protein in the form of a fusion protein comprising a foreign peptide having specific binding affinity to a binding partner selected from the target molecule or a surface of the magnetic particle, the method comprising the steps of:
(a) incubating the affinity separation means with the sample for a period of time sufficient to allow the filamentous phage bind to any target molecule present in the sample;
(b) separating the affinity separation means from the sample; and
(c) eluting the bound target molecule from the affinity separation means.

2. A method as claimed in Claim 1 in which filamentous phage is functionalised with a hydrophilic polymer such as PEG.

3. A method as claimed in Claim 1 or 2, in which the foreign peptide is a silica-binding peptide, and the magnetic particle comprises, or is functionalised or coated with, silica, and wherein the filamentous phage is coupled to the magnetic particle by means of a specific binding reaction between the silica-binding peptide forming part of the filamentous phage and the silica coated or functionalised magnetic bead.

4. A method as claimed in any preceding Claim, in which the foreign peptide is a polyhistidine tag, and the magnetic particle comprises, or is functionalised or coated with, nickel or cobalt, and wherein the filamentous phage is coupled to the magnetic particle by means of a specific binding reaction between the polyhistidine tag forming part of the filamentous phage and the nickel or cobalt forming part of the magnetic particle.

5. A method as claimed in any preceding Claim in which the filamentous phage is genetically engineered to express (a) a first major coat protein in the form of a fusion protein comprising the major coat protein and a peptide having specific binding affinity to the target molecule, and (b) a second major coat protein in the form of a fusion protein comprising the major coat protein and a peptide having specific binding affinity to a binding partner forming part of the magnetic particle.

6. A method as claimed in any of Claims 1 or 2 in which the magnetic particle is coupled to the filamentous phage by means of an antibody, wherein the antibody is chemically or physically coupled to the magnetic particle and has specific binding affinity to at least one coat protein of the filamentous phage selected from pIII, pVIII, pVI, pVII, and pIX.

7. A method according to any of claims 1 or 2 in which the filamentous phage is chemically coupled to the magnetic particle by means of ethyl-dimethyl-aminopropylcarbodiimide and N-hydroxy-succinimide (EDC/NHS) cross-linking which couples basic amino acid residues on a major coat protein of the phage with the magnetic particle.

8. A method according to any of Claims 1 or 2 in which the filamentous phage is coupled to the magnetic particle by coupling between biotin and a biotin bindning partner, in which the surface of the magnetic particle is functionalised with a biotin binding partner and basic amino acids of a coat protein of the phage are biotinylated.

9. An affinity separation means suitable for the selective purification of a target molecule and comprising a filamentous phage functionalised magnetic particle in which the filamentous phage is genetically engineered to express at least one coat protein in the form of a fusion protein comprising a foreign peptide having specific binding affinity to a binding partner selected from the target molecule or a surface of the magnetic particle.

10. An affinity separation means as claimed in Claim 9 in which the phage is functionalised with a hydrophilic polymer such as PEG.

11. An affinity separation means as claimed in Claim 9 or 10 in which the foreign peptide has specific binding affinity for a target molecule selected from a peptide, polypeptide, protein, protein complex, recombinant protein, antibody, antibody fragment, an immunoglobulin selected from the group IgG, IgM, IgA, IgD, IgE, chimeric IgG, human IgG, humanised IgG, a polynucleotide, sugar, and a polysaccharide.

12. An affinity separation means as claimed in Claim 9 or 10 in which foreign peptide is a silica binding peptide, and wherein the surface of the magnetic particle is modified with silica, wherein the magnetic particle is functionalised with phage through interaction between the silica binding peptide forming part of the phage and the silica forming part of the magnetic bead.

13. An affinity separation means as claimed in any of Claim 9 to 11, in which the foreign peptide is a polyhistidine tag, and the magnetic particle comprises, or is functionalised or coated with, nickel or cobalt, and wherein the filamentous phage is coupled to the magnetic particle by means of a specific binding reaction between the polyhistidine tag forming part of the filamentous phage and the nickel or cobalt forming part of the magnetic particle.

14. An affinity separation means as claimed in any of Claims 9 to 13 in which the filamentous phage is genetically engineered to express (a) a first coat protein in the form of a fusion protein comprising a foreign peptide having specific binding affinity to the target molecule, and (b) a second coat protein in the form of a fusion protein comprising a foreign peptide having specific binding affinity to a binding partner forming part of the magnetic particle.

15. A filamentous phage genetically engineered to express at least one coat protein in the form of a fusion protein comprising a silica binding peptide.
